Europäisches Patentamt

European Patent Office

Office européen des brevets

(11) Publication number: **0 444 649 A2**

# EUROPEAN PATENT APPLICATION

(21) Application number: **91102956.9**

(51) Int. Cl.5: **C12Q 1/68**

(22) Date of filing: **27.02.91**

(30) Priority: **27.02.90 AR 316268**

(43) Date of publication of application:
**04.09.91 Bulletin 91/36**

(84) Designated Contracting States:
**DE ES FR GB IT NL**

(71) Applicant: **AGRILAB BIOTECHNOLOGY LTD.**
**26, Tel Hai Street**
**Raanana 43405(IL)**

(72) Inventor: **Burochik, Moises**
**Gomez de Fonseca 225**
**Buenos Aires(AR)**
Inventor: **Haim,.Liliana**
**J.J. Paso 1139**
**1640 Martinez PCIA De Buenos Aires(AR)**

(74) Representative: **Josif, Albert et al**
**Baaderstrasse 3**
**W-8000 München 5(DE)**

(54) **Procedure for the detection of plant pathogens under field conditions and a diagnostic kit for its application.**

(57) A process for the detection of plant pathogens performed totally or in part under field conditions, comprising selecting a plant tissue presumably containing at least one phytopathogenic agent, localizing a sample dot from the plant tissue on a support membrane to immobilize the nucleic acids of the phytopathogenic agents, fixing said nucleic acids onto the membrane, hybridizing the nucleic acids with a hybridization solution containing an auxiliary reactive group, washing said membrane to remove the probe which did not hybridize, treating said membrane with a blocking solution, treating the membrane with a reagent to bind an enzyme to the auxiliary reactive group, washing said membrane with a second washing solution, treating the membrane with a chromogenic substrate solution capable of producing an insoluble colored compound, and detecting the presence of the change in color with the naked eye; and a diagnostic kit for carrying out said procedure.

EP 0 444 649 A2

## 1. INTRODUCTION

The primary matter of the present Patent of Invention is a procedure for the detection of phytopathogenic agents in infected plants, as well as in their insect vectors and in symptomatically unaffected host plants. The most remarkable feature of this procedure is that it can be performed totally or in part under field conditions, which will make this procedure to be preferred over the previously known techniques, in most applications. An additional important feature of the procedure here presented, is that it allows an early diagnosis of plant diseases caused by phytopathogens, which can be detected before the appearance of visible symptoms. Another additional matter of the present invention is the set of reagents and devices with which the procedure for the detection of phytopathogens is carried out, totally or in part under field conditions.

The procedure here presented consists, essentially, in choosing a plant tissue (e.g. leaves, fruits, stems, roots, etc.) presumably infected with the phytopathogen, taken from the infected plant or from a plant not showing disease symptoms but grown in areas in which the disease in prevalent, or in taking insect vectors from the field.

Afterwards, in applying one or more samples of the above tissues or insects onto an appropriate support membrane, followed by a fixation step by which the nucleic acids in the samples become immobilized on the membrane. Next, the above nucleic acids are contacted with a "cold-labeled" probe under conditions favouring hybridization of complementary nucleic acids. The probe here mentioned is a DNA molecule containing, at least: a) a sequence (1000 to 3000 nucleotides long), complementary to the phytopathogen nucleic acids, and b) a "cold" label, which is non radioactive, nor carcinogenic, nor mutagenic nor otherwise toxic, and which will allow further chemical (enzymatic) detection. After hybridization, the membrane is washed, blocked to prevent non-specific binding, and treated with one or more solutions with reagents which bind to the label of the probe resulting in the formation of successive stable complexes, one of them containing a conjugated enzyme.

Finally, the membrane is treated with an appropriate chromogenic substrate which, in the presence of the above enzyme, produces and insoluble compound of a predetermined color. The appearance of this color on the sample spots, which clearly differs from the background color observed in areas of non-hybridized samples, constitutes the indication which allows the detection of the infected samples. All the above steps of the procedure were here developed to a point of great simplicity for their execution under field conditions. This is an important feature for crop management when quick decisions are required, e.g., when it is intended to prevent the spread of an incipient plant disease in the field.

The procedure here presented allows for interruptions or stopping points. Therefore, the execution of the procedure can be started at the field and completed elsewhere, in a laboratory location. This is a convenient situation, as highly trained personnel, involved in qualified laboratory work is not required to perform the first steps of the procedure, which are carried out at the field.

## 2. Background of the invention.

The rational and integrated management of the different factors that affect the yield of commercially important crops, has become a critical tool for agriculture economics. Decisions involving such management demand precise, quick and detailed information.

Among the different kinds of information required, a relevant one concerns the detection and diagnosis of the pathogen-borne diseases affecting the crops.

When this information is available at the early stages, i.e. before visible symptoms appear, it is then possible to evaluate, for example, the potential threat of a pest, its probable or actual incidence, and the most adequate steps intended to avoid or reduce its possible impact, as well as the cost/benefit ratios involved in each of the different treatment alternatives.

Since the precise and sensitive diagnosis is frequently more critical in the case of plant virus diseases than for those caused by other phytopathogenic agents (e.g. fungi, for which there exist, and are currently applied, broad spectrum control agents), the crop protection strategies against plant viruses are oriented towards:

a) the eradication of the sources of infection (insect vector, infected plants, virus hosts or reservoires),

b) to prevent or delay the dissemination of the virus through control of its vectors (when they exist), or

c) incorporation of virus resistance or tolerance into the plants, by genetic manipulations.

The existing methods for the detection of virus in plants or insect vectors depend, among others, from the following factors:

a) the presence, in the plants or insect vectors under analysis, of specific viral proteins which can be detected by immunological methods, thereby indicating the presence of the virus.

b) the immunogenic ability of the mentioned specific viral proteins to produce, when used to immunize the adequate animals, sera containing antibodies against those proteins at adequate titers for the further detection of the mentioned proteins by immunological methods.

c) the presence in the natural phytopathogenic viral entity of certain proteins, usually the virus coat proteins. Consequently, if the phytopathogenic agents are naked viruses, lacking coat protein viroids, they will not be detected by the immunologic methods currently in use.

d) the utilization of radioactive atoms in some of the reagents being used.

e) the use of complex and expensive laboratory equipment. The following diagnostic methods are currently used to detect viruses in plants and insect vectors.

Biological methods. They consist in the transfer of the virus from the samples under test to adequate "indicator" plants and the visual scoring on these of symptoms of the disease.

The transfer (infection) is done by mechanic inoculation (tissue abrasion of the indicator plant and application of the material to be tested on the wound thus produced) or by means of the natural infected vector or by grafting the sample tissue onto the indicator plant.

Although these methods are widely used, they have several drawbacks such as: the need of controlled greenhouses, high labour requirement, the danger of contamination and propagation of unknown or exotic viruses, as well as the long time (sometimes several weeks) for the development of visible typical symptoms to diagnose the disease.

Other complications of the method are that, occasionally the particular host genotype of the indicator plant may affect the production of symptoms or the inoculation may be difficult.

Electron microscopy: The direct visualization of the viral particles with the electron microscope, though possible, is quite imprecise since it is frequently difficult to distinguish between the particles of different viruses. Moreover, many viruses are present in the plants at very low concentrations, making it difficult to prepare the samples for electron microscopy.

These inconveniences, that hamper the presymptomatic detection of plant viruses, can be partially solved by the use of the Immunosorbent Electron Microscopy (ISEM) techniques. With these techniques, viruses are specifically identified because they form immunocomplexes with antibodies directed against their coat proteins, which have been labeled with heavy atoms for their detection with the electron microscope.

Although these techniques using the immunological properties of the viral proteins, are more precise than visual detection, they share with it several important disadvantages, involved in the use of the electron microscope, including: high equipment cost, the need for qualified operators and the slow throughput in the processing of the samples.

Immunologic procedures: The in vitro immunodiagnostic methods are more practical than the ones aforementioned, and they consequently seem to dominate among the biotechnology-derived diagnostic tests. They are based on the preparation of antibodies against appropriate viral antigens (usually the viral coat protein(s)) and the specific reaction of these antibodies with the viruses present in the sample.

This reaction may be performed through various analytical configurations, among which the one called enzyme-linked immunosorbent assay (ELISA), is widely used because of its simplicity, sensitivity and low cost.

Although very convenient, the immunological procedures, including ELISA, have some disadvantages: a) in an increasing number of plant diseases, viroids have been shown to be the causative agent; viroids are small nucleic acids (as compared with the viruses) and "naked" (without coat); as they lack coat protein, viroids are unable to elicit the necessary antibodies to develop immunological detection methods; b) some plant viruses can also occur as naked nucleic acids,(particle-deficient viruses), with the same limitations as viroids (see point a) in this enumeration) for their detection through immunological methods; c) in some cases, during the purification of the viruses (necessary for the further immunization of the animals from which the antibodies will be obtained) it is difficult to thoroughly preserve the native state and/or composition of the antigenic proteins; as a result, the preparations used for the immunization process will have variable protein composition, which will yield antibodies preparations difficult to standardize, making the detection of the natural viruses imprecise; d) many viruses are difficult to purify to the necessary degree and amount to obtain antisera of the required quality; e) many plant viruses are poor immunogens (high titer antisera cannot be obtained).

The above difficulties of the immunological methods are such that their application is not feasible in some cases (points a), b) and eventually d) of the above enumeration), or will lack and adequate precision (points c), e) and eventually d), in the above enumeration).

3

Molecular hybridization of nucleic acids.

In the last few years, new diagnostic methods have been developed in phytopathology in general, applied in particular to the detection of phytopathogenic viruses in plants and insect vectors.

The above methods are based on the hybridization of nucleic acids, which allow the unequivocal detection of a given phytopathogenic entity, particularly viruses, through a very specific reaction of its genetic material, with a labeled probe, i.e. the formation of a duplex, hybrid molecular complex.

The utilization of molecular hybridization with labeled probes as the basis for the specific detection of nucleic acids is one of the most significant advances in diagnostic techniques derived from the application of biotechnology.

The probe must be synthesized in such a way that it will only hybridize with the target complementary nucleic acid to be detected. When the hybrid is formed, which means a positive identification, this is then evidenced through a second functional portion of the probe, here identified as the "label" (the first functional portion is the probe's unique nucleotide sequence, which is essential in the hybridization reaction).

The "label" has particular reactive properties which will reveal the presence of the probe in the hybrid, i.e., the presence of the hybrid itself. This will mean that the nucleic acid being assayed was present in the sample under test.

In most cases, the "label" is a radioactive atom of the probe so that a positive hybridization reaction is shown by autoradiography or by other suitable technique for the detection of radioactive elements.

These procedures involve the handling of radioactive substances, which can only be performed by specially trained personnel. It also requires costly and specific equipment and auxiliary elements, the adequate facilities approved by the authority that regulate and control the use of radioactive elements, careful precautions during work, safe disposal of residues and also involve risks for the operator.

Several publications, dealing with the above procedures, have appeared in the scientific literature in the last years. For instance, in a publication (R.A. Owens and T.O. Diener, 1981), the authors report on the use of a radioactive DNA probe hybridization assay for the autoradiographic detection of a plant disease caused by a viroid. Since the above publication, however, only some of the steps of the detection procedure, e.g. the preparation of the probes, have been improved for routine diagnosis. The inconveniences derived from the use of radioactive probes were not solved, and the extraction of the viral nucleic acids required, in general, relatively complicated laboratory equipment.

A noteworthy advance in the latter area has been reported recently (N. Navot et al, 1989), involving the introduction of a very simple sampling technique, which can be performed at the field by personnel not specially trained. This technique consists essentially in squashing the plant tissue onto a membrane, by pressing with a hard object. This technique has been adopted in the present invention, among the preferred ways for sample application.

In view of the above inconveniences (i.e. the use of radioactive labels), "cold" non-radioactive labels have also been developed for the detection of the hybrids formed with the probe. Some of these non-radioactive techniques can reach sensitivity levels comparable to those obtained with radioactive labels. Some of these techniques, in particular, because of their high sensitivity and simplicity, are adopted as a part of the present invention, in a particular and novel application. These techniques involve reagents which are neither toxic nor carcinogenic and are therefore quite safe for the operator.

Examples of publications dealing with the above techniques can be found in the literature, and are briefly outlined below. In one of these examples (N. Habili et al, 1987), plant extracts are prepared by methods which can hardly be considered for field use: leaves were crushed in a roller press (sap extractor), or powdered in liquid nitrogen and crushed in a ball-bearing device, or homogenized in a glass rod homogenizer, and a crude nucleic acids extract is obtained.

In another report (H.E. Hopp et al, 1988), samples are prepared also by laborious methods: clarified supernatants from plant tissues obtained by homogenization of liquid nitrogen or dry ice-frozen tissues in a precooled mortar, are further processed for RNA extracts preparation by conventional extraction and precipitation steps. In a simpler technique, plant tissues are extracted in plastic bags, but the extract is submitted to similar RNA extracts preparation protocols. All the above procedures are quite difficult to apply if a high number of samples are to be tested at one time.

Other references from the literature can also be given, dealing with non-radioactive probes for the detection of plant viruses and viroids, which again relay in laborious laboratory techniques for sample preparation (J.L. McInnes et al, 1989; M. Eweida et al, 1989).

Useful references dealing with the detection of viruses by nucleic acids hybridization with non-radioactive probes, in fields other than Phytopathology, can also be found in the literature (Ch. Clavel et al, 1989; M. Inoue et al, 1989).

Summarizing, from the above publications it seems evident that methods are known for the assay of plant tissues for virus detection, in which the samples can be obtained under field conditions (N. Navot et al, 1989) but relaying on the use of radioactive probes. On the other hand, the use of non-radioactive probes for plant virus detection has been reported (M. Eweida et al, 1989; N. Habili et al, 1987; H.E. Hopp et al, 1988; J.L. McInnes et al, 1989), but associated with sampling techniques which do not lend themselves to be used in the field. Finally, in other areas of Biology, diagnostic methods based on nucleic acid hybridization with non-radioactive probes have been reported, but they call for sampling techniques which are not applicable in Phytopathology.

As will be shown below, the procedure of the present invention significantly improves on the previous art, not only by combining techniques which were not previously used together, but also because each step has been developed to a point which allows to perform the whole test under field conditions, or to perform the first steps (including the sampling) at the field, and to complete the test in a laboratory.

The merit of the present invention resides in that it combines the scientific advances of Molecular Biology with simple technical concepts, in the development of a procedure and a diagnostic kit for the detection of phytopathogens. This combination, which is what is claimed in the present Patent of Invention, allows the detection of phytopathogens with sensitivity and specificity levels higher than those of previously known procedures. In addition, the procedure presented here can be performed under field conditions, it does not require special laboratory equipment or trained personnel, and allows to obtain the results in a short time and at early stages of an infection, all of which constitute great advantages when compared with the previous art.

As stated above, it is evident that nucleic acids probes can be used for the diagnosis of plant viral diseases, which, because of the precise and extensive complementarity requirements of the hybridization reactions, result in an efficient and accurate means for disease monitoring in field crops. ·

Through the method of the present invention, which is based in the detection and identification of the nucleic acids of the pathogen (viruses, viroids, microorganisms and even invertebrate parasites), diagnostic methods are now available for use in agriculture, which are reliable, sensitive, quick and specific, also satisfying the requirement of an early detection and even allowing the diagnostics of phytopathogenic agents in general, either unicellular or multicellular organisms (e.g. nematodes), under field conditions.

The method which is the object of the present invention relies on the specific molecular interaction of a special reagent (the probe) with the genetic makeup of the virus and therefore it does not require the presence, in the plants or insects under analysis, of specific viral proteins, neither does it depend on the integrity of such proteins; it neither depends on the biological complexity of the viral entity (since it can detect viroids), nor on the utilization of radioactive elements or complex laborotory equipment. Another advantage of this method, which stresses the difference with most of the current methods, is that it can detect, in a specific fashion, the several variants of a given phytopathogenic organism.

This is important in many cases where the different genotypes of the same phytopathogenic agent have a different importance with regard to their ability to cause disease, susceptible hosts, etc. It must be pointed out that, although the present description will mainly make reference to the diagnosis of phytopathogenic viruses and viroids, the diagnostic concept referred to in the present invention is of general validity.

It must be understood, therefore, that the particular description herein presented does not represent any limitation to the scope of the present invention, which is extended to include the diagnosis of plant diseases caused by invertebrate parasites and microorganisms (including fungi and bacteria).

In the diagnostic method of the present invention, the identification is based on the hybridization of the nucleic acids of the sample with a labeled probe.

It is the main matter of the present invention, a procedure for the detection of plant pathogens, performed totally or in part under field conditions, characterized by the following stages:

a) To select a plant tissue presumably containing a population of at least one phytopathogenic agent.

b) To obtain, form the selected tissue, a sample localized as a dot or dot-blot onto an appropriate blotting or support membrane, having a filtering layer to which the nucleic acids of the phytopathogenic agent from the said population can be immobilized.

c) To bind (fix) onto the said support membrane, in a stable manner, the nucleic acids of the phytopathogenic agent from the said population, eventually present in the localized sample.

d) To bring into close contact the sample localized onto the support membrane with a hybridization solution containing at least one probe, complementary to the nucleic acids of the at least one phytopathogenic agent, the said probe being labeled with an appropriate labeling group.

e) To wash the support membrane with a first washing solution, until all non-hybridized probe is removed.

f) To treat the support membrane with a blocking solution.

g) To bind to the labeling group (from the at least one probe) of the hybrid molecules, at least one reagent contained in at least one solution, by treating the support membrane with said at least one solution of the reagent, so that an enzyme results bound to the complex, the said enzyme being able to act upon a chromogenic substrate producing an insoluble colored compound.

h) To wash the support membrane with a second washing solution, until all non reacted reagents are removed.

i) To visualize the formation of hybrid molecules in the localized sample, by treating the support membrane with a solution of a chromogenic substrate capable of producing, in the presence of the said enzyme, an insoluble colored compound, so that a change in color is visualized with the naked eye on the localized sample, detecting the presence of the population of the at least one phytopathogenic agent in the selected plant tissue.

An accesory object of the present invention is an assembly of reagents and other components, for the application of the above procedure which is the principal object, characterized by containing, at least:

a) The said support membrane, having a filtering layer capable of immobilizing the nucleic acids of the phytopathogenic agents.

b) At least one hybridization solution containing at least one probe labeled with an appropriate labeling group, complementary to the nucleic acids of at least one phytopathogenic agent.

c) The said first washing solution.

d) A blocking powder diluent.

e) A blocking powder to be dissolved in the blocking powder diluent right before its use, in order to obtain the mentioned blocking solution.

f) At least one solution of at least one reagent containing an enzyme, capable of binding to the hybrid molecules presumably formed.

g) The said second washing solution.

h) The said solution of a chromogenic substrate.

The most remarkable feature of the procedure here presented, namely that it can be wholly performed under field conditions, was achieved through research on laboratory methods, as well as by combining known techniques, e.g. the "squash-blot" already mentioned (N. Navot et al, 1989), with protocols adapted from reports involving the use of non-radioactive probes.

There are situations in which it could be more convenient to perform the test only in part under field conditions. Although this would be possible in several ways, two particular cases are considered most convenient, here indicated by reference of the description of the procedure above:

1) to perform steps a, b, and c, in the field, and the rest of the procedure in a distant or centralized laboratory, or 2) to perform all steps from a, to e, in the field, and to complete the procedure in the laboratory. in the first case, the membrane with the applied samples do not require additional steps for completion of the test, whereas in the second case, after step e, the membrane should be dried and kept dry during its transfer to the laboratory (at room temperature), where it should be re-hydrated for continuation of the procedure. Although normal waiting times are tolerated in both cases, in the second case it is convenient that the membrane be shipped under conditions similar to those obtained in a laboratory dessicator.

The first stopping point is advisable when decisions are to be made at the laboratory. In this case, the performer will be able to select the probes to be tested, e.g. the less specific ones (for the detection of several genetically close phytopathogenic agents) or the highly specific (for the discrimination of closely related variants from the same species).

On the other hand, there are situations in which it would be advisable to perform the hybridization (steps d, and e,) in the field. This would be the case when the manager needs to take a decision quickly at the field, in response to other direct observations of the crop (appearance, symptoms ), or when plant health protection agencies keep for themselves the decision (and the responsibility) of the probes selection, e.g. when inspecting plant materials seeking entry permits into a country.

The present invention claims a methodology for the application of the above concepts, that allows to separate the sampling step, which can be performed directly on the field by applying the samples to the adequate support membranes, from the further analytical process which can be carried out at a distant laboratory receiving the mentioned support membranes containing the samples.

Such operative separation allows to supply a diagnostic service, performed at a reference laboratory, with the adequate samplings being obtained at distant areas by the field operators. These operators will use a "sampling equipment" with all the elements required to obtain the samples through simple field methods.

The concept of "sampling equipment" is possible because of the simplicity of the methods for sample application and is facilitated by the format and physical characteristics of the device on which these samples are applied, namely, the support membranes. These characteristics allow the use of regular mail to send to the laboratory the elements representative of a large number of plants or insects, in a small size parcel.

It is also possible for the operators at the field to perform the hybridization step, and to send the treated membrane to the laboratory where the visualization steps would be performed.

It must be anticipated that, although the examples of the present invention here described pertain to a certain number of viral entities as detected by the method being claimed, these examples must be considered as "models" of general application, since one of the main features of the present invention - which is also claimed - is its general applicability to the detection of other viruses and viroids, as well as of other phytopathogenic microorganisms.

Consequently, the present invention refers not only to a series of procedures and products for the detection of certain phytopathogenic viral entities but also, and particularly, to a concept which can be the basis for more novel diagnostic methods for use in agriculture.

3. Description of the Procedure - Characteristics.

The diagnostic method referred to in the present invention, differs from the other existing methods (see Point 2. Background of the Invention) in the following characteristics:

A. Simplicity of operation. This means that it can be used under "field conditions", not requiring special laboratory equipment. In some of the examples to be described, the whole procedure can be carried out at room temperature, independently from laboratory equipment. The search for operative simplicity is an obvious trend in diagnostic methodologies as it promotes its acceptance and diffusion and render the most advanced technologies available to wider sector of the target markets.

B. Simplicity in the obtention of samples. This is a feature in which the characteristics detailed in point A) above are more evident and useful. In most cases where the present invention will be of application and, particularly, in the examples to be described in the corresponding section, the samples (both from plant tissues and insect vectors) are applied by squeezing them with a light pressure using a hard object, or by the simple imprint of a fresh piece of newly cut tissue, directly onto the surface of the detection support.

This technique is known as "squash-blot" (sample impregnation by squashing or blotting) in the context of the present invention, and constitutes an originality that is also claimed among the techniques of this method.

The possibility of applying the "squash-blot" technique derives essentially from two fundamental characteristics of the method of the present invention (and is thus included among the claims of the present invention), namely, its high sensitivity and the conceptual basis of the detection event (i.e., the specific hybridization of nucleic acids).

These characteristics, which are not present in current commercial methodologies for the detection of plant pathogens, turn unnecessary many other methods for the preparation of samples, which are tedious, complicated, and in general require great care and professional handling with specific equipment and reagents (preparation of homogenized tissues, isolation of nucleic acids).

Although the technique for sample application here called "squash-blot" is one of the main characteristics claimed in the present invention, the subsequent methodological steps are wholly compatible with other methods of sample application which can be used at the operator's option.

These compatible techniques can be, amongh others:

1) The obtention of cellular fluid by squashing or mashing the plant tissue in a device or container, and the application of an adequate volume of this fluid (filtered, centrifuged or as is) onto the detection membrane.

This operation must be done quickly to avoid the possible degradation of the relevant nucleic acids, produced by the enzymes which are released when the structure of the tissue is broken.

This is particularly important in the case of the detection of viruses or viroids, specially when they are present in very small quantities, as plant tissues are rich in nucleases that rapidly degrade the viral RNAs or DNAs, unless special precautions are not taken beforehand. In comparison with the "squash blot", this technique for the preparation of samples requires more time and a larger number of laboratory elements, as the cross-contamination between samples must be avoided when processed simultaneously.

2) The preparation of an homogenate of the plant tissue or insect vectors, utilizing a solution at the adquate pH and containing agents which immediately denature (turn inactive) the nucleic acids-degrading enzymes. This homogenate (filtered, centrifuged or as is), can be applied as before onto the detection membrane.

Obviously, time, manipulation and laboratory elements required for this technique are still greater than those demanded by the proceeding one.

3) Either if techniques 1) or 2) above are used, the plant tissue extracts or homogenates can subsequently be purified to a certain extent, yielding solutions (which can be applied onto the detection membrane), from which some impurities have been removed. Eventually, even the nucleic acids can be fractionated so as to obtain the relevant fraction. These procedures are more laborious than the former ones.

C. Configuration.

The format adopted for the present invention corresponds to the "method for the hybridization of nucleic acids on a solid support". In this technique, a specially suited membrane (or membrane filter) function as the "detection support". The materials constituting such support include a nitrocellulose, nylon or modified nylon, surface, with the ability to bind (immobilize) the nucleic acids in a stable manner. Membrane filters suitable for the proper execution of the procedure claimed in this invention are commercially available from several manufacturers, and are supplied in various sizes, porosities and binding properties.

The immobilization is carried out in practice on a limited area on the surface of the membrane (called "dot blot", impregnation or blot of a spot or limited area). This area is then the site where the further processes will take place on the sample and on which the results of the tests will be observed at the end of the procedure.

Since the nucleic acids of each sample are individually fixed on the different "dots" and there is no possibility for diffusion, mixing or contamination among them, which could affect their individuality, integrity and correct and unequivocal localization, the detection support-membrane can be manipulated as a unit, thus greatly simplifying the analytical protocol when many samples are processed simultaneously.

The area occupied by the material of each sample applied to the detection support (which is known as "dot" in the literature), and on which the whole analytical process and visualization of the results take place, may be very small, e.g. down to 3-5 mm2 (i.e., 2-3 mm diameter). Thus it is possible to analyze a large number of samples (for example, several hundreds of them) with small pieces of detection support and without the need for many other laboratory ware, as the samples remain permanently separated and are easily located throughout the whole process up to the visualization of the results.

When the number of samples is small, a dot size of 7-8 mm2 is more convenient.

The solid support configuration also makes possible:

1) To pre-treat the membranes with adequate solutions to avoid the degradation of the nucleic acids of the sample.

2) To send a membrane containing the samples (for example by mail) to a laboratory that will carry out the analysis. This allows the application of a large number of samples in the field through a simple and quick method and their further analysis in some other place.

This possibility is afforded by the great stability of the samples immobilized on the membrane, which can be stored for several months (at room temperature) before being analyzed, without losing the integrity of the nucleic acids fixed onto it (the only precaution being to avoid exposure of the membrane to high humidity).

3) To send (for example by mail) a membrane containing the hybridized samples to a centralized or reference laboratory, for the further development of the visualization process, i.e., its treatment with the one or more reagents with affinity for the probe labeling group or for a molecule previously bound to the labeling group, one of the said reagents being conjugated to an enzyme, and its further treatment with a solution of a chromogenic substrate.

In this way, a substantial portion of the procedure is left for the laboratory, where a number of membranes can be processed together (if the label of the different probes is the same), leaving the selection of the probes (as well as the responsibility for such a seleciton) to the field operators. These field operators can make their choice based on conditions not known to the laboratory (e.g., an increase in the population of insect vectors). With this mode of operation, the only precautions in the handling of the membranes will be to dry them after the post-hybridization wash, to ship them in a tight container (with a dessicant to prevent them from becoming moist), and to re-hydrate them for continuation of the procedure.

4) Easy registration and documentation of the results (the color on the dots indicates the result of each sample) by simply photocopying the membrane.

8

D. Use of non Radioactive Reagents. The analytical procedure referred to in the present invention is based on the detection of the molecular hybrids formed by the nucleic acids of the samples with the specific, labeled probe. The probe bears a chemical, non radioactive label, and the visualization of the hybrids is done by means of non radioactive reagents.

As non radioactive elements are used, the method of the present invention is: safe for the operator; applicable in any laboratory under field conditions with no special precautions; free from hazardous wastes; independent from facilities such as freezers, dark room, etc., and elements such as X-ray films, intensifying screens, etc.; quicker (as compared with the auto-radiography).

A high sensitivity is achieved in the procedure here presented, even without the use of novel, improved techniques for the preparation of the probes which are described below (dimers of the probe, homo-polynucleotide tailing of the probe). This high sensitivity, which is comparable to that obtained in procedures employing radioactive probes and autoradiography, results essentially from the enzymatic step of the procedure here presented. By acting as catalysts, enzymes are able to turn over high amounts of substrate, thus "amplifying" the positive signal.

E. Flexibility - Versatility. A main element of the present invention is the DNA probe. This probe includes nucleotide sequences which are complementary to the nucleic acids to be detected. Since the composition of the probe (the selected complementary sequence), its size (within certain limits) and the hybridization parameters, can all be modified, diverse methodologies can thus be designed. For example, probes can be designed to detect the presence of viruses and other microorganisms constituting genetically related groups, taking advantage of the sequence homologies of the nucleic acids within the members of the group. In this case, the test will determine if some member(s) of the group is(are) present in the sample, but will not identify it(them).

On the other hand, the procedure can be made so stringent that the test will distinguish between slightly genetically different variants or will determine the relationship (degree of homology) among different viruses or microorganisms or among strains of a given species (differential probes). Also, the objective of the test could be the detection in the sample of at least a single member of a group of non genetically related phytopathogenic viruses or microorganisms. In this situation, all detecting probes can be used simultaneously in a single test (a poly-probe). The presence of only one of the members of the group will suffice to give a positive result. This is a desirable situation when the objective of the test is to verify the absence of any of a number of viruses or microorganisms, e.g. the compliance with a given plant health specification or with the requirements of a health qualification or certification.

Not only the detection specificity can be varied by changing the composition (selected nucleotide sequence) and size of the probe. Within the present invention it is also possible to modify these latter parameters (composition and size) in order to achieve an increased detection sensitivity, as will be described under point 4 below, with regard to two techniques which have been successfully developed: the use of probe dimers (resulting in an increase in the label-bearing sites per hybrid-forming probe molecules), and the artificial addition of certain homo-polynucleotide chains at one end of the probe (with a consequent increase in sites for potential labeling), here also presented as "C-tailing", because the chain added is constituted by cytosine residues.

F. Wide Use. The hybridization of complementary strands of nucleic acids is at the very foundation of modern Biology. The possession of genetic material constituted by some kind of nucleic acids is a universal characteristic of living beings. The genetic makeup is specific for each species, variety, type or strain under consideration. In this context, "specific" means the possession of a unique nucleic acid sequence.

A nucleic acid probe (the "reagent") is essentially a nucleic acid chain able to form a hybrid with another nucleic acid strand (the "sample"), provided the sequence of the latter is complementary to the one present in the probe.

Therefore, the hybridization of nucleic acids is a biological identification concept of general validity.

It includes viruses and microorganisms as well as higher organisms like invertebrates. Moreover, it refers not only to the genetic information contained in the genome of the organism in question but also to the genetic elements (nucleic acids) which are "dispensable" in the sense that, although not essential for the replication of the organism, their presence confers it some evolutive advantages (survival in certain habitats, for example).

Some of these dispensable, non-genomic elements (for example, plasmids in bacteria) have been found to be associated with very important characteristics of the organisms that possess them (e.g., virulence, pathogenicity). In these cases, the relevant information required in the diagnostic will refer primarily to the detection of such accesory genetic elements, which, being nuceic acids, are also within the scope of the capabilities of the method of the present invention.

Many viruses, microorganisms and invertebrates are important agricultural pests and, consequently, the method claimed by the present invention has a wide range of applications for the diagnosis in agriculture. The main concept and the methodological scheme that can be carried out for all those applications is basically similar (that is: hybridization, labeled non-radiactive probe, label detection reagents, detection by enzymatic reaction).

It will only be required to use the adequate specific probe and to adjust every detail of the methodological procedure. For this reason, the primary claim of the present invention is the detection and identification of phytopathogenic agents in agriculture through techniques of molecular hybridization of nucleic acids with specific non radioactive probes, which can be performed, totally or in part under field conditions, because of the features of the methods for sample preparation.

4. Description of the Product.

The product embodied in the diagnostic method referred to in the present invention is a set of reagents and other elements which are assembled in an essentially complete, self-contained set (kit).

A general description of the product includes:

A) The labeled specific DNA probes: These are deoxy-ribonucleotide chains (namely, poly-deoxy-ribonucleotides) whose nucleotide sequences are specific and precisely complementary to adequate portions of the viral nucleic acids or, in general, to the nucleic acids of the phytopathogenic entities to be detected. This structural complementarity at the molecular level is the very basis of both, the identification reaction (in which the hybridization is the first and decisive step) and of its specificity, since a given "probe" will only hybridize with the nucleic acids which it has been specifically designed to detect.

Along with its specific sequence (equivalent to the specific arrangement of the coded letters of a unique message), the polydeoxy-ribonucleotide molecules contain, in adequate places, certain auxiliary reactive groups (the "labels") which will then allow the visualization of the identification complex (the formation of the "hybrid"). If the hybridization has been performed on a solid support configuration, the label will remain on the detection support only if the hybrid has been formed (positive identification). If the hybrid is not formed (meaning the absence of the nucleic acid the detection of which is being tested), the probe will not be retained on the detection support and, through its removal, the label it carries will also be removed.

In this context, we call "detection support" to an adequate device (a supporting membrane of an appropriate material), the characteristics and application of which are described below. It will suffice to mention here that the different steps of the detection method are carried out on it.

Neither the probes employed in this method nor the labels they carry contain radioactive atoms. The reactive groups in the labels will allow to visualize their presence by means of reactions which do not involve the use of radioactivity.

From the several labelling techniques available, two different types have been included in the present invention:

1) The biotinylation, which consists in the introduction of biotin molecules in the poly-deoxy-ribonucleotide chain of the probe. This introduction can be done through different procedures of the molecular biology domain, involving incorporation of biotinylated derivatives of some natural deoxy-ribonucleotides into the DNA chain of the probe.

We have used the following procedures: a) the technique denominated "nick translation" (stepwise degradation and immediate resynthesis of one of the DNA strands with the adequate enzymes, in which the intact strand acts as the template; during the resynthesis, some natural deoxy-ribonucleotides are replaced by their biotinylated derivatives and incorporated into the newly synthesized chain, carrying with them the biotin label); b) the synthesis of oligonucleotides (also employing the biotinylated derivatives of the deoxy-ribonucleotides, for the synthesis of oligonucleotide mixtures containing different sequences complementary to portions of the genome of the phytopathogenic entities to be detected); or c) the direct covalent binding to the probe of biotin or of an adequate reactive biotin derivative (for example photobiotin, a biotin derivative which can be activated by irradiation with visible light generating a highly reactive radical which covalently binds biotin to the polynucleotide chain of the probe).

2) The sulfonation, which consists in the introduction of a sulfone group into some cytosine residues of the polynucleotide chain of the probe. This sulfonation is performed in such a way that the cytosine derivative, $N^4$ methoxy-5,6-dihydrocytosine-6-sulfonate, is stabilized in the chain of the probe.

We have been able to increase the number of labeling groups introduced into the probe, and hence the sensitivity for the detection, by increasing the number of target sites (i.e. the cytosine residues) for the labeling reaction. Obviously, the addtion of cytosine residues cannot be made within the hybridizing sequence, as this would destroy its complementarity with the nucleic acids we wish to detect. Instead, cytosine residues can be added to one end of the probe molecule, as a poly-cytosine tail ("poly-C tailing"), without affecting the hybridiztion behavior of the probe. Poly-C tailing, which can be performed by enzymatic methods, will increase the number of cytosine residues per probe molecule, thereby increasing the number of labeling groups it carries and hence the sensitivity for the detection of these groups.

B. The detection supports: These are membranes, also called "membrane filters", constructed with nitrocellulose or nylon (the latter sometimes modified), with the ability to bind and immobilize nucleic acids (DNA or RNA). The above materials are presented as homogeneous films or as layers on an inert structural support.

When a sample of the material to be tested (for example: a plant tissue extract obtained with saline solutions or directly the exudate resulting from a fresh cut, crushing or compression of plant tissues or organs) is applied to the detection support, the nucleic acids are immediately bound to the membrane. After a simple treatment with heat, microwaves, ultraviolet light or alkaline solutions, depending on the membrane, this binding becomes very stable.

In this way, the detection support will contain, in well defined areas, the "dots" bearing the nucleic acids of the sample. As this binding is very stable, the remaining steps of the analytical method are carried out directly on the detection support as a unit, the results being finally visualized on each dot.

The dots corresponding to each sample can occupy small areas (e.g. 3-5 mm2). Therefore, a membrane of a small size (for example 10 cm x 10 cm), can accommodate a large number of individual samples.

In addition, the membranes used as detection supports can be pretreated with appropriate reagents, whenever this is convenient for the particular purposes of the detection protocol.

C. The label detection reagents. These reagents will bind to the label, allowing the direct visualization of the hybrid. The nature of these reagents will depend on the type of label carried by the probe, and are described here in connection with the two types of labels mentioned above.

The biotin label is very efficiently recognized through the binding of avidin or, alternatively of its microbial analog, streptavidin. Both proteins have a high affinity for biotin, to which they bind strongly. These proteins have four binding sites for biotin per molecule, so that when one of them is occupied by the biotin label of the probe (which is immobilized with the hybrid on the detection support), they will still have other vacant sites for biotin binding.

These free sites can then bind additional biotin molecules, to which an adequate enzyme (for example, alkaline phosphatase) has been previously conjugated through covalent bonds. The strong affinity for biotin of the avidin or streptavidin already present in the complex on the support, will cause the binding of the "biotinylated" enzyme (or "biotin-enzyme conjugate") to the said complex. The formation of this complex can now be detected because it contains an enzyme, whose presence can be visualized through its action on adequate substrates, as explained below.

From the above description it is evident that the use of the two mentioned reagents (avidin or streptavidin and biotinylated enzyme) has the purpose of introducing the enzyme into the complex initiated by the formation of the hybrid. This can also be achieved by the use of only one reagent, i.e. an avidin-enzyme conjugate (or streptavidin-enzyme conjugate).

In this case, the affinity of the biotin for the avidin or streptavidin will also result in the incorporation of the enzyme into the detection complex. Both reagent systems, the one with two components (avidin or streptavidin and biotinylated enzyme), or the one with only one component (avidin-enzyme or streptavidin-enzyme conjugates), can be applied in the procedure of the present invention.

It is also possible to design other alternatives, for example, by means of anti-avidin (or streptavidin) antibodies conjugated with the enzyme, which can replace the second reagent in the two components system explained above.

The sulfonated cytosine label is recognized through an immunoenzymatic system of the "sandwich" type. This system requires two reagents for the incorporation of the enzyme (which will allow the visualization) into the hybrid containing the labeled probe.

These two reagents are:

1) A mouse monoclonal antibody, directed against DNA modified by sulfonation: This antibody will bind to the hybrid at the sulfonated groups on the probe.

2) To the complex thus formed is then added a preparation of goat anti-mouse immunoglobulin G (IgG) (polyclonal antibody), to which the desired enzyme, for example alkaline phosphatase, has been covalently conjugated (i.e. by means of the bifunctional reagent glutaraldehyde).

This IgG (goat) anti-mouse:enzyme conjugate, will then bind (through a classic antigen-antibody reaction, in which the mouse monoclonal antibody is the "antigen", and the IgG (goat) anti-mouse is the "antibody") with immunological specificity, to the mouse monoclonal antibody previously immobilized on the detection support.

Alternatively, an innovation in the detection of the sulfonated cytosine label has also been developed, which consists in the utilization of a single immunoenzymatic reagent: A mouse monoclonal antibody (directed against DNA modified by sulfonation) conjugated with the enzyme. This reagent incorporates the enzyme without the need of a second antibody, affording a more simple methodology and significant technical advantages.

We have developed another improvement for the detection of this label, namely the above referred "poly-cytosine tailing", which consists in the artificial addition of a number of cytosine residues to an end of the probe. As the number of cytosine residues per probe molecule are thus increased, so are the sites (sulfonated cytosines) capable of binding the label detection reagents, which results in an increased detection sensitivity.

Either by employing the biotin label (biotinylated probe) or the sulfonated cytosine label (sulfonated probe), formation of the hybrid on the detection support and the binding of the above reagents will result in the immobilization of an enzyme to the hybrid.

Therefore, the reagents for the detection of the label, described in this section, are complemented with a substrate of the enzyme, which will not depend on the kind of label but on the chosen enzyme.

In the present invention, alkaline phosphatase is used as an example, with nitro-blue tetrazolium (NBT) and 5-Bromo-4-Chloro-3-indolylphosphate (BCIP) as chromogenic stubstrates. The action of the enzyme on these compounds results in the formation of an insoluble colored precipitate on the area of a positive (label-containing) dot on the detection support. Other enzymes, different from the one indicated above can also be used in the present invention (e.g. peroxidase, acid phosphatase), which will require the use of their corresponding substrates.

Also, the enzyme mentioned in the example (alkaline phosphatase), can be visualized by means of different chromogenic substrates, e.g. a combination of acid 3-hydroxy-2-naphtoic-2,4 dimethylanilide phosphate and the colorant 1 chloro-2-methylbenzene diazonium salt, among others.

Whatever the system used for probe labeling, in all cases the labeling groups are the sites for the critical binding of the detection reagents. It follows that this detection can be enhanced by the use of dimers of the probe, i.e. single molecules in which two copies of the probe are linked together: when one of these copies hybridizes to the target nucleic acid, a double number of labeling groups (compared to a "single" probe molecule) results immobilized. An enhanced sensitivity for the detection of the hybrids can therefore be expected, which has been proved in the development of the present invention.

D. Auxiliary solutions. Several steps of the procedure of the present invention require the employment of the following auxiliary solutions:

1) Sample extraction solution. In most cases where the present invention is applicable, the samples in which the presence of viruses or other phytopathogenic agents is to be tested can be obtained and applied by pressing plant tissues or insect vectors directly onto the detection support, without the need of ad hoc extraction procedures, as explained in the description of the method involved in the present invention.

Nevertheless, the operator may choose to extract the tissues or insects under test with adequate solutions for the solubilization of the nucleic acids of the phytopathogenic agents to be determined, carefully preventing their enzymatic degradation, which might otherwise ensue through the action of the enzymes released when the structure of the tissue is broken.

In such cases, the method proposed in the present invention employs an extraction solution constituted by an adequate saline solution, for example, but not exclusively, a buffer and denaturing solution, containing guanidine isothiocyanate and 2-mercapto-ethanol, of the following composition:

```
Guanidine isothiocyanate ..... 3 to 5   M

2-Mercaptoethanol ...........     50 mM

EDTA .........................     20 mM

MES  .........................     20 mM

                      pH :       7.0
```

MES: 2-(N-morpholino)ethanesulfonic acid.

2) Prehybridization solution. For the application of the procedure of the present invention, a prehybridization solution has been used, which adequately prepares the detection support for the hybridization step. The composition of this solution is:

```
2.a)

Formamide ...  50 %    SDS ................. 1     %

NaCl ........ 450 mM    Sodium Pyrophosphate   0.001 %

NaPO4H2 ..... 25 mM    PAES ................ 0.1   %



        EDTA ........ 2.5 mM

        SDS: Sodium Dodecyl Sulfate.

        PAES: Sodium Polyanetholesulfonate.
```

Alternatively, a prehybridization solution of the following composition can also be used:

2.b) 5M guanidine thiocyanate-0.1 M EDTA (1).... 6 ml

20 x SSC solution (2) ..................... 1 "

15 % poly (acrylic) acid (3) solution ..... 1 "

20 % sarcosine solution ................... 0.5 "

10 mg/ml denatured sonicated salmon sperm

DNA solution ........................... 100 ul

Complete with distilled water to .......... 10 ml

pH : 7.5·

(1) Stringency of hybridization (degree of complementarity required for hybridization) can be varied by modulating the final concentration of guanidine thiocyanate from 2M (high stringency) to 4M (low stringency).

(2) The composition of the 20 x SSC solution is:

NaCl .................. 3 M

Trisodium citrate ..... 0.3 M

pH : 7.0 (adjust with 1 M HCl).

(3) Molecular weight 90,000 (available from Aldrich Chemical Co.). Neutralized with HONa pellets, diluted to 15% and sterilized (autoclave) before use.

3) For the application of the procedure of the present invention, a hybridization solution of the following composition had been used:

```
Formamide ...  50 %    SDS ...................... 1     %

NaCl ........ 450 mM    Sodium Pyrophosphate ..... 0.001 %

NaPO4H2 ..... 25 mM     PAES .................... 0.1   %

EDTA ........ 2.5 mM    Labeled probe ...0.1 to 1 ug/ml.
```

Alternatively, a hybridization solution of the following composition can also be used:

```
5 M guanidine thiocyanate - 0.1 M EDTA (1) ....... 6    ml.

20 x SSC solution (2) ............................ 1    "

15 % poly (acrylic) acid (3) solution ........... 1    "

20 % sarcosine solution ......................... 0.5 "

10 mg/ml denatured sonicated salmon sperm

   DNA solution ................................ 100 ul.

Labeled probe ...........................0.2 to 10 ug.

Complete with distilled water to ...........  10   ml.

                                      pH: 7.5
```

(1) (2) (3) See notes under point 2.b.) above.

4) Washing solutions-1 (post-hybridization). For the application of the procedure of the present invention, two washing solutions-1 (post-hybridization) of the following composition have been used:

```
a) NaCl ........... 150 mM  b) NaCl ............... 15  mM

   Trisodium citrate 15 mM     Trisodium citrate .. 1.5 mM

   SDS ............ 0.1%       SDS ............... 0.1 %
```

Both solutions above, a) and b), are adjusted to pH:7.0. Alternatively, a post-hybridization washing solution-1 of the following composition can also be used:

```
c) NaCl .................. 300 mM

   Trisodium citrate ....  30 mM

   Tween 20 ............. 0.1 %

             pH: 7.0
```

5) Blocking solution. In the procedure of the present invention, the detection reagents used after the hybridization step are prevented from non-specific binding to the detection support. To this end, the following reagent is used: Blocking diluent. Composition:

```
NaCl .............. 25 mM      Tween 20 ....... 0.3 %

Tris. HCl (pH 7.5) 50 mM   (*)Heparin (Na) ... 3.5 mg/ml.

EDTA ............. 1 mM                       (500 u/ml)
```

(*) A "Blocking Solution without Heparin" is also prepared, in which this component is omitted. PAES (see 4.D.2.a) could also be used instead of Heparin.

Immediately before use, 0.3 g of a blocking powder are dissolved for each ml of this solution. The blocking powder has the following composition:

```
Skimmed milk ......................... 91 g.

Bovine serum albumin (Fraction V) ....  9 g.
```

6) Washing solution-2 (post-antibodies). For the application of the present procedure, a washing solution-2 (post-antibodies) of the following composition is used:

```
NaCl .................. 0.5 M

Tween 20 .............. 0.1 %
```

7) Substrate diluent solution. For the application of the present procedure, a substrate diluent solution of the following composition is used:

```
Tris. ClH .................. 100 mM   pH: 9.5

NaCl ....................... 100 mM

MgCl2 ......................   5 mM
```

8) Chromogenic substrate solution. For the application of the present procedure, a chromogenic substrate solution is used, which is made up as follows:
To a solution of the following composition:

Tris. ClH .......... 100 mM pH:9.5

NaCl .............. 100 mM

MgCl$_2$ ............... 5 mM, the following components are

added per ml:

3 mg of a mixture of nitro-blue-tetrazolium + glucose (1 part + 9 parts by weight, respectively), and

4 ul of a solution of 5-bromo-4-chloro-3-indolyl phosphate in N,N-dimethylformamide (50 mg/ml).

9) Nucleic acids binding solutions. When the procedure of the present invention is used with membranes constructed with modified nylon, the nucleic acids are bound to the membrane after sample application, by treating successively with the following solutions:
a) Alkaline solution: 0.5 M HONa
b) Neutralizing solution:

Tris. HCl ....... 0.1 M

NaCl ............ 1  M

pH : 7.5

5. Detailed Description of the Invention.

A general description of the detection method referred to in the present invention is detailed below. In the relevant steps, different alternatives are also described which are also included among the claims of the present invention.

5.1. Sample preparation.
5.1.1. (Alternative 1). Squash-blot (direct impregnation of the detection support membrane).
A small portion of fresh plant or insect tissue is squeezed by means of a hard object (for example a glass rod) onto a small area of the supporting membrane (made of nitrocellulose, nylon or modified nylon). In many cases, the cutting of fresh plant tissue (leaf, stem, fruit, root, tuber, micro-propagation material, callus, etc.), allows the direct application of sap exudated through the area of the cut, without squeezing the tissue.
With the squash-blot technique, the area occupied by each sample can be very easily delimited by means of a thin and rigid sheet used as a "mask" (for example a plastic sheet) which has been perforated to create sampling "windows", e.g. circles of 2 to 8 mm diameter. This sheet is placed over the membrane so that the "window" openings expose the areas onto which the samples are to be applied.
A portion of plant tissue is placed over the "window" area and squeezed against the membrane by means of a hard object. The "mask" will limit the area on the membrane impregnated by the sample.
After squeezing the tissue sample, any remaining plant or insect material is carefully removed with tweezers or a small spatula.
The support membrane must be handled with great care to avoid contaminations. Disposable gloves and tweezers should be used. If the genetic material of the pathogen under test is constituted by double stranded DNA, the samples applied onto the membrane are first treated with 0.5 M NaOH-2M NaCl during 5 minutes, and later neutralized with 1 M Tris. HCl-1.5 or 2.0 M NaCl.Ph:7.5.
If the genetic material of the phytopathogenic entity to be detected is constituted by single stranded DNA (e.g. a member of the geminivirus group), the above treatment can be omitted. When the analysis deals with genetic material constituted by RNA (as is the case with most of the phytopathogenic viruses), the membrane must be pretreated (before applying the sample) by wetting it with a solution containing 0.4-0.5 % sodium dodecyl sulfate and 50-100 ug/ml of proteinase K. The samples applied by the squash-blot technique described above are very stable and can be stored at room temperature for several months without losing their ability to perform in a reliable test, provided they are not exposed to high humidity conditions.

5.1.2. (Alternative 2). Direct dot-blot of sap ("dot" application of sap or liquid exudate).

A small portion of fresh plant or insect tissue is squeezed (e.g. in a test tube) with a hard object to obtain a sap, juice or liquid exudate from which a small volume (for example 3 ul per dot, 10 ul/cm2 of membrane, etc.) is applied onto the membrane.

Precautions in the handling of the membrane and its previous treatments are similar to those indicated under Alternative 1 (point 5.1.1.).

5.1.3. (Alternative 3). Juice dot-blot ("dot" application of partially clarified juice).

Small portions of fresh plant tissue or a few insects are crushed and ground with a hard object in an adequate container (for example, a small plastic centrifuge tube) and briefly centrifuged (e.g. 5 minutes at 3000 rpm), to obtain a partially clarified supernatant.

A small volume of this supernatant is applied onto the detection support, as indicated in the above alternative (point 5.1.2.).

5.1.4. (Alternative 4). Extract dot-blot ("dot" application of an extract).

Small portions of fresh plant tissue or a few insects are crushed or ground with a hard object in an adequate container (i.e. a small plastic centrifuge tube) and extracted with a solution prepared as per point 4.D.1 of the present invention. Other extraction solutions can also be used, provided they quickly denature the enzymes eventually present, which can degrade the nucleic acids of the sample.

The crude extract thus obtained is briefly centrifuged and the supernatant is then applied onto the membrane as indicated in the former alternative (point 5.1.3.).

5.1.5. (Alternative 5). Dot-blot of purified nucleic acids(1) ("dot" application of purified nucleic acids).

The procedure is similar to that indicated under Alternative 4, (point 5.1.4.) up to the stage in which the supernatant of the crude extract is obtained.

This supernatant is then extracted with one volume of phenol-chloroform-isoamylicalcohol (25:24:1); to the aqueous phase, 0.2 volumes of 1 M acetic acid and 0.7 volumes of ethanol are added and the mixture is kept during 2 hours at -18/-20° C. This mixture is centrifuged and the precipitate is resuspended in the following solution:

$$\text{Tris-HCl} \ldots\ldots\ldots\ldots\ldots \quad 10 \quad \text{mM}$$

$$\text{EDTA} \ldots\ldots\ldots\ldots\ldots\ldots \quad 1 \quad \text{mM}$$
$$\text{pH} : 7.8 - 8.0$$

From the solution of the nucleic acids so obtained, a small aliquot is then applied to the membrane, as indicated in point 5.1.3.

The care exercised in handling the membrane as well as its pre-treatments, are as indicated under point 5.1.1.

5.1.6. (Alternative 6). Dot-blot of purified nucleic acids(2) ("dot" application of purified nucleic acids).

Steps are similar to those under alternative 4, (point 5.1.4.) up to the stage in which the supernatant of the crude extract is obtained.

This supernatant is then purified by means of an ion exchange chromatography minicolumn, using an adequate resin for anion exchange.

By selecting the resin, a solution containing the desired non-fractionated nucleic acids (DNA or RNA) can be obtained. This solution is now the sample, a small aliquot of which is applied onto the membrane as indicated under point 5.1.3. The care in handling the membrane, as well as other steps (e.g. the treatment in the case of double stranded DNA) are as indicated under the alternative described in point 5.1.1.

5.1.7. (Comments on Alternatives 2 to 6). Dot-blots. All the alternatives for sample application involving Dot-blot techniques (Alternatives 2 to 6, here described in order of increasing handling requirements) differ from Alternative 1, (Squash-blot) in that they require more manipulation of the sample.

Although Alternative 1 is the most simple and can be performed under field conditions, the other alternatives can also be simplified and thus made less time-consuming by utilizing devices for simultaneous and multiple sample processing commercially available ("manifold" type apparatus and "multichannel" pipettes).

5.2. Immobilization of the nucleic acids on the membrane.

There are cases in which it is not necessary to perform any additional treatment to fix the nucleic acids to the detection support membrane: the binding will take place during the application of the sample, assisted by the nature of the membrane (e.g., nylon and modified nylon) and by the nature and concentration of the nucleic acids to be immobilized.

On the other hand, a strong and permanent binding of the nucleic acids to the membrane can also be obtained by means of some of the following methods:

5.2.1. Irradiation with ultraviolet light (long wave) during 2-5 minutes ( for nylon membranes).

5.2.2. Baking (with or without vacuum) at 65°C to 80°C, during 30 minutes to 2 hours (for nylon or nitrocellulose membranes).

5.2.3. Baking in a microwave oven for apprcx. 3 minutes.

5.2.4. Treatment with an alkaline solution and further neutralization (for modified nylon membranes).

Before performing any of the above operations, the membranes bearing the samples must be dried (at room temperature or with a hair dryer).

When submitted to baking, the membranes must be wrapped with filter paper.

Once dry, the membranes containing the samples (either or not previously baked or irradiated) can be stored for several months at room temperature (for example in a file chest) until their further analysis.

This allows the sample to be safely sent by regular mail to associated laboratories, plant health control authorities, etc.

5.3. Pre-hybridization.

The membrane containing the immobilized samples is wetted with the pre-hybridization solution during 20-30 minutes at 37°C to 42°C. As will be explained in some examples, this step may be omitted in many cases, thus simplifying the methodology.

5.4. Hybridization.

5.4.1. Hybridization is performed by placing the membrane and an adequate volume of the solution of the labeled probe, in a polyethylene bag or similar device which can be easily closed (by folding or sealing the open end of the bag) and allows to manipulate the solution of the labeled probe in order to uniformly distribute it over the membrane.

5.4.2. Alternatively, a piece of polyethylene film on which the solution of the labeled probe is distributed, can also be used. The membrane is then placed over this solution (the side bearing the samples facing the solution) and covered with another polyethylene film. A rigid sheet is then placed over the latter and a weight is placed on top to press the whole assembly.

The sizes of the pieces of polyethylene film are such as to adequately cover the membrane. The weight is used to achieve a thorough contact of the membrane with the solution of the labeled probe. The rigid sheet will distribute the compression uniformly.

5.4.3. Whichever of the above alternatives is used, the objective is to place the samples applied onto the membrane (which has been previously drained to remove the excess of pre-hybridization solution), in effective contact with the solution of the labeled probe.

The diluent of this solution is the pre-hybridization solution (or other adequate hybridization mixture), and it contains the most convenient concentration of the probe, which has been optimized in each case through prior testing. Immediately before its use, the solution of the labeled probe must be denatured by immersing it in a boiling water bath during 5 minutes, after which the solution is rapidly chilled in an ice bath.

The volume of labeled probe solution to be used in the hybridization depends on the nature of the membrane and must be such that it will soak its surface entirely, avoiding air pockets as well as an unnecessary excess.

Volumes of approximately 10 to 100 ul/cm$^2$ of membrane are normally used.

The solution of the labeled probe, which is one of the main elements of the product and the procedure of the present invention, must be used in aliquots adequate for their complete use in each test, keeping the remaining without any manipulation before its use. The solution of the labeled probe is stable for several months if stored at 4°C.

The hybridization is performed by incubating the membrane imbibed with the labeled probe solution, during 30 minutes to 2 hours (or more, in some cases up to 12 hours) at 25°C to 65°C. conditions for hybridization must be optimized in each case.

5.5. Post-hybridization wash.

The membranes are washed in plastic trays, using washing solution (3-7 ml per cm2 of membrane). The membrane must be washed to remove the probe that has not hybridized with the nucleic acids of the sample.

Washing is performed twice with Washing Solution 1 (a) and then twice with Washing Solution 1 (b), each time during 5-30 minutes at 25°C - 65°C. The tray is moved to shake the washing solutions that bathe the membrane. The membrane is thoroughly drained after each wash.

5.6. Blocking.

The membrane, thoroughly drained from the last wash, is soaked in a volume of blocking solution (for example, 50 ul/cm2 of membrane), during 1 hour at room temperature (25°C to 30°C).

5.7. Visualization of the hybrids.

The method used for the visualization of the hybrids depends on the kind of label of the probe. Also, for a given label, there are different alternatives which are better explained for their claim as parts of the present invention, in the following examples.

Example 1. Detection of potato viruses PVX, PVY, PLRV and Viroid PSTV.

1. The membranes (of nylon 0.45 um pore size in this example) were pre-treated by moistening with an aqueous solution of sodium dodecyl sulfate (0.4-0.5%) containing Proteinase K (50-100 ug/ml). Leaf samples of both healthy and infected plants were applied as indicated in point 5.1.1. (Squash-blot), or in point 5.1.2. (Direct juice dot-blot), or in point 5.1.3. (Juice dot-blot), or in point 5.1.4. (Dot-blot of extract), or in point 5.1.5. (Dot-blot of purified nucleic acids-1), or in point 5.1.6.(Dot-blot of purified nucleic acids-2). All these techniques proved to be adequate.

Tuber samples, also from healthy and infected plants, were applied employing the same techniques as with the leaves. Several zones from the tubers were tested, with the area of the rose end, a few milimeters deep from the external surface (close to the vascular system), being the more convenient for viruses detection. A piece of this area was cut, and the moist, fresh tissue applied onto the membrane with a slight pressure. From both, leaves and tubers, parallel samples (i.e. from the same plant or tuber) were applied on four similar membranes, following a sequential order that allowed their further unequivocal identification.

2. The membranes bearing the samples (for example 100 samples in one membrane of 10 cm x 10 cm) were let to air-dry during 10 minutes, wrapped in filter paper and baked for 2 hours at 80°C.

3. The membranes were soaked in pre-hybridization solution (see 4.D.2.) during 30 minutes at 40°C and they were further Processed separately. Each membrane was individually hybridized with its specific labeled probe as explained in 5.4.2., after which they were washed as indicated in 5.5.

4. The labeled probes used in this example have been prepared as described below.

The potato viruses PVXc and PVY°, obtained from the International Potato Center (IPC, Lima, Peru), were multiplied and purified according to the usual methods of the art (P. Gugerli, 1978, 1979). The synthesis of the respective cDNA "libraries" from the viral RNAs were done as per published methods (U. Gubler and B.J. Hoffman, 1983).

The cDNAs thus obtained were cloned in the pBS(+) phagemid vectors (Stratagene, 1988).

Positive clones were detected with the usual methods of the art, in particular, by hybridization with the corresponding $^{32}$P-labeled viral RNAs.

The probes employed in the procedure of the present invention were selected on the basis of insert sizes and their behaviour in hybridization experiments with extracts from tissues of plants infected with the corresponding viruses. Similar methods were utilized for the virus PLRV and for the viroid PSTV.

Single strand DNA probes complementary of the viral nucleic acids were obtained by the technique described in detail by the supplier of the vector (Stratagene, 1988). The technique suggested by the ion exchange supplier (Diagen, 1988) for phage M13 single strand DNA, was also successfully employed. The resulting single strand pBS(-) DNAs, were labeled by the methods described below.

 a) with photobiotin (A.C. Forster et al, 1985),
 b) by sulfonation (Orgenics, 1989).

Another technique successfully employed consisted in the preparation of a double strand DNA from pBS phagemid, which was purified by known procedures (H.D. Birnboim, 1983), and labeled by biotin-14-dATP incorporation (J.J. Leary et al, 1983; B.R.L., 1988). DNAse concentration was 4 pg/ul and incubation time was 2 hours at 15° C. Under these conditions the biotinylated nucleotide substituted about 25% of the Adenine residues and the average size of the resulting nick-translated DNA fragments was approximately 500 nucleotides.

In the case of the viroid PSTV (constituted by a small circular, single-stranded RNA molecule of 359 nucleotides), a complete cDNA clone was obtained, from which, using the same techniques described above, the corresponding single stranded DNAs were obtained. From these, the one corresponding to the right polarity was selected, as the probe to hybridize with the viroid RNA.

Except for the case of PSTV, whose insert size is determined by the viroid genome size (359 nucleotides), the viruses cloned inserts had sizes ranging from 1000 to 3500 nucleotides approximately.

5. As continuation of point 3 above, after hybridization, the membranes were washed (always separately for each virus tested) and blocked as indicated in point 5.6 (Section 5), using the appropriate volume of blocking solution (Section 3.D.5.).

6. For the visualization of the hybrids formed with the nonradioactive labeled probes, the respective commercially available techniques and reagents were used (B.R.L., 1988, for the biotinylated probes; Orgenics, 1989, for the sulfonated probes) with slight modifications.

After the blocking step, the membranes were treated either with a streptavidin-alkaline phosphatase conjugate (1 ug/ul) for biotinylated probes, or with a specific monoclonal antibody solution (dilution 1:250, in a recently prepared blocking solution, without polyanetholesulfonate, Orgenics) for the sulfonated probes.

After incubating at 37° C during 30 minutes, the membranes were washed three times, for 2 to 3 minutes each time, with a washing solution (NaCl 0.1 M; Tris.HCl 0.1 M; MgCl$_2$ 3mM; Tween 20 0.1%; pH:7.5), at room temperature.

In the case of sulfonated probes, a step was added where the membranes, well drained after the first washing, were treated (always separately) with an adequate volume (i.e. 50 ul/cm2 of membrane) of blocking solution without heparin containing anti-mouse hyperimmune goat serum, whose immunoglobulin G (IgG) has been conjugated with alkaline phosphatase (Orgenics). A dilution of this serum in the blocking solution without Heparin of about 1:250 (expressed in volume of serum solution: volume of blocking solution without heparin), was used.

The membranes were incubated with this solution of IgG-alkaline phosphatase conjugate during 1 hour at room temperature (25° C to 30° C).

After this, the membranes were washed five times with gentle shaking, during 2 to 3 minutes each time, with washing solution-2 (post-antibodies, Section 3.D.6).

The following steps allowed the visualization of the results, using the action of the alkaline phosphatase enzyme, which resulted incorporated to the membrane in the areas of the samples which hybridized positively with the probes.

The membranes were washed (always separately) with substrate diluent solution (section 3.D.7.), and were treated with an adequate volume (e.g. 50 ul/cm2 of membrane) of chromogenic substrate solution (section 3.D.8), prepared immediately prior to its use, in a dimly illuminated place. The addition of this solution to the membranes was done under dim lighting conditions.

The membranes were left in the dark for 15 to 60 minutes, or until a light purple-blue color become visible in the areas of the membrane not containing samples.

After obtaining a satisfactory color development, the nylon membranes were washed to stop the reaction with alcohol 96%, distilled water, then with a diluted solution of commercial chlorine bleach for domestic use (approximate concentration: 20 g/l of active chlorine) and finally again with distilled water.

The treatment with the bleaching solution will remove interfering colors derived from the plant tissue, and could be omitted when such colors are absent (for example, with potato tubers).

With the nitrocellulose membranes, the reaction was stopped by washing them directly with distilled water.

The results were read on the wet membranes.

The presence of the viruses was evidenced by a purple-blue dot in the area of the sample. When the viruses were absent, the sample area only presented a very light coloration, similar to that of the membrane surface not bearing samples. The membrane was dried with filter paper and photocopied for a permanent record of the results.

The conclusions drawn from this example, with regard to the different techniques for the application of the samples used here, allowed us to verify the efficacy of the most simple techniques for use in the filed, that is, the "squash blot" (Point 5.1.1.) and the "dot blot" of juice (Point 5.1.2). The other techniques applied (Points 5.1.3 to 5.1.6) were also adequate but more complex. Therefore, they would only be justified in cases where a higher level of sensitivity is desired (as they allow to increase the amount of sampled plant tissue). This higher sensitivity level is usually unnecessary for diagnostic purposes. On the other hand, these latter techniques can also be applied even without the need for facilities of a conventional laboratory, thanks to the current availability of the adequate elements, such as syringe filters, ion exchange minicolumns adaptable to automatic pipets, multiple dot-blot application devices, etc.

As previously indicated, two new techniques have been tested successfully to improve the sensitivity of virus detection referred to in this example (see points a) and b) below).

a) The use of "dimers" of the probes, obtained by linking the 3' end with the 5' end of the corresponding "monomeric" probes, thus doubling the number of "labels" per each hybrid formed. For the obtention of the dimers, the double strand DNA probe is used, which is cloned (inserted) into a plasmid at a high probe/plasmid ratio (e.g., 20:1); recombinant clones are identified through the usual techniques of the art, and the plasmids with the expected size for the insertion of 2 copies of the probe in a single vector molecule are selected (for example by agarose gel electrophoresis); and b) the addition of a polycytosine tail (for the case of the detection of sulfonated cytosines) to 3' end of the probe, thus artificially increasing the number of modified residues. This was done by treating the probe with a restriction endonuclease generating protruding 3' ends in a double strand DNA (for example, Pst I or Sac 1) and further incorporating cytosine residues to that end (with dCTP, deoxy-cytidine triphosphate in the presence of enzyme "terminal transferase"), until the desired number of cytosine residues was incorporated.

Both techniques, particularly when they were successively applied to the same probe, resulted in a significant increase of the detection sensitivity.

Example 2.

Monitoring, early diagnosis and prevention or limitation of the attack of the Tomato Yellow Leaf Curl Virus (TYLCV), through the use of a detection method based in the molecular hybridization of nucleic acids, claimed in the present invention.

1. In the present example, a specific probe for the detection of the TYLCV in plants and insect vectors is assumed to be available, as well as an adequate methodology to carry out such detection with the features of the present invention, i.e. ability to process a great number of samples in a short time, under field conditions and with high specificity and sensitivity.

2. The stems of the tomato plants under epidemiologic control were cut with a sharp instrument and some sap was applied in each numbered place of a detection support-membrane. A grid of 0.5 cm side squares was previously printed with a common pencil.

This technique allowed to apply more than 150 samples (from as many plants) in approximately 2 hours.

3. In the field under control, the procedure claimed in the present invention allowed to diagnose the disease in 60% of the plants, 10 days before the appearance of visible symptoms. Four days before the symptoms appeared, the TYLCV specific probe allowed to detect the presence of said virus in the whole population of the plants of the field under investigation.

The use of these techniques of early diagnosis would allow the necessary time to eradicate the infected plants in time, thus reducing the virus reservoirs available as inocula by the dissemination of the insect vectors.

4. The monitoring of the presence of virus in the population of insects in the field under control, showed that in a period of approximately 45 days, the percentage of whiteflies carrying detectable amounts of virus (through the methods of the present invention) raised from 27% to 66%. This increase was related to the increase in the number of infected plants and with the severity of the symptoms of the disease. The verification of such correlations can supply the necessary information to indicate the opportunity and time for the application of insecticides, thus significantly reducing the impact of the disease in the field.

5. As an example of a viable strategy, derived from the above point, the insects potentially carrying the TYLCV virus may be tested about two or three times a week as from the start of the seeding period, within or around the field that is being monitored. An increase in the proportion of insect vectors can be used as an indication to start or intensify the application of the insecticides. Since the populations of whiteflies can migrate in an unpredictable way, this monitoring must be done at a regional, national or international scale, which can be easily done due to the format used in the methodology of the present invention: the support-membranes containing the squashes of the insects tested can be shipped by mail to a central laboratory, where results will be issued back to the field, permitting to detect, geographically localize and evaluate the potential for infection of the insects population.

This will allow each producer to take in time the decisions needed to reduce the economic impact of the disease, to avoid dissemination of a possible epidemic, etc. In this way, some examples on the performance of the method and of the reagents and devices for its application have been described as part of the present invention, the range for protection of the invention being mainly defined by the claim clauses detailed hereunder.

According to a preferred embodiment of the procedure for the detection of plant pathogens of the invention, the enzyme in step g) is alkaline phosphatase, said solution of chromogenic substrate useful in step i) and containing, at least, nitro-blue-tetrazolium and 5-bromo-4-chloro-3-indolyl phosphate in a diluent, is prepared immediately before step i) under dim lighting conditions, said treatment of the detection support membrane in said solution of chromogenic substrate is performed in the dark during 15 to 60 minutes, washing it afterwards in a first step with alcohol 96% and in a second step with distilled water, and visualing on the wet membrane the appearance of a purple blue coloration detecting the population of at least one phytopathogenic agent.

According to a further preferred embodiment of the procedure for the detection of plant pathogens, of this invention, after the second step of washing with distilled water of said membrane, a third step follows consisting in washing with commercial chlorine bleaching solution in an approximate concentration of 20 g/l active chlorine and a fourth step of washing with distilled water. Preferably said second washing step is done with a solution of Tween 20 at 0.1% concentration.

Still according to a preferred embodiment of the procedure for the detection of plant pathogens, in step d) the detection support membrane is put into close contact with a hybridization solution, wetting it completely and incubating for a period between 20 minutes and 12 hours, at a temperature between 25 and 65°C.

According to a preferred embodiment of the set of reagents for the application of the procedure according to this invention the probe of the hybridization solution b) is obtained from the double stranded DNA monomeric probe, which is cloned in the following step into a plasmid vector at a high probe/plasmid ratio, the recombinant clones being identified and selected in the following step as the plasmids presenting inserts of the size corresponding to two copies of the probe into the vector molecule.

When the probe has said additional tail of several sulfonated cytosines covalently linked to one end of the probe DNA strand, said covalent binding is obtained by treating the probe with a restriction endonuclease generating protruding 3' ends in a double strand DNA and, in the following step, incorporating cytosine in said ends with deoxycytidine triphosphate in the presence of the enzyme "terminal transferase".

Preferably the set of reagents according to the invention also comprises the mentioned pH buffer/extraction solution which denatures the enzymes degrading the nucleic acids, and constituted by guanidine isothiocynate 3 to 5 M, 2-Mercaptoethanol 50 mM, EDTA 20 mM, 2-(N-morpholino)ethane-sulfonic acid 20 mM, pH:7.0. Furthermore, the set of reagents also comprises the mentioned solution for the pretreatment of the detection support membrane constituted by an aqueous solution of sodium dodecyl sulfate 0.4 to 0.5% and proteinase K 50 to 100 $\mu$g/ml.

Still more preferably, the set of reagents also comprises the mentioned prehybridization solution of the following composition: formamide 50%, sodium chloride 450 mM, sodium acid phosphate 25 mM, EDTA 2.5 mM, sodium dodecyl sulfate 1%, sodium pyrophosphate 0.001%, sodium polyanetholesulfonate 0.1 %.

LITERATURE CITED

(1) R.A. Owens, T.O. Diener, "Sensitive and Rapid Diagnosis of Potato Spindle Tuber Viroid Disease by Nucleic Acid Hybridization", Science (1981) 213:670-672.

(2) N. Navot, R. Ber and H. Czosnek, "Rapid Detection of Tomato Yellow Leaf Curl Virus in Squashes of Plants and Insect Vectors", Phytopathology (1989) 79:562-568.

(3) N. Habili, J.L. Mc Innes and R.H. Symons, "Nonradioactive, photobiotin-labelled DNA probes for the routine diagnosis of barley yellow dwarf virus", J. Virol. Methods, (1987) 16:225-237.

(4) H.E. Hopp, L. Giavedoni, M.A. Mandel, A. Arese, B.Orman, F. Bravo Almonacid, H.N. Torres and A.N. Mentaberry, "Biotinylated nucleic acid hybridization probes for potato virus detection", Arch. Virol.(1988) 103: 231-241.

(5) J.L. Mc Innes, N. Habili and R.H. Symons, "Nonradioactive, photobiotin-labelled DNA probes for routine diagnosis of viroids in plant extracts", J. Virol. Methods, (1989) 23:299-312.

(6) M. Eweida, T.L. Sit and M.G. Abouhaidar, "Molecular cloning of the genome of the carlavirus potato virus S: biotinylated RNA transcripts for virus detection in crude potato extracts", Ann. appl. Biol. (1989) 115:253-261.

(7) M. Inoue, M.A. Duggan, D.I. Robertson and V. Chang-Poon, "Non-isotopic detection of HPV DNA in cervical smears using dot-blot hybridization", J.Virol. Methods, (1989) 26: 159-170.

(8) Ch. Clavel, M. Doco-Fenzi, A. Lallemand, I. Binninger and Ph. Birembaut, "Détection par hybridation in situ des papillomavirus avec des sondes marquées par sulfonation", Ann. Pathol. (1989) 9 (N° 2): 137-139.

(9) P. Gugerli, "The detection of two potato viruses by enzymed-linked immunosorbent assay (ELISA)", Phytopathol. Z., (1978) 92: 51-56.

(10) P. Gugerli, "Potato virus A and Potato Leafroll Virus: Purification, Antiserum Production and Serological Detection in Potato and Test Plants by Enzyme-linked Immunosorbent Assay (ELISA)"; Phytopathol. Z, (1979) 96: 97-107.

(11) U. Gubler and B.J. Hoffman, "A simple and very efficient method for generating cDNA libraries", Gene, (1983) 25: 263-269.

(12) Stratagene, 1989 Product Catalog, Stratagene, La Jolla, California, U.S.A.

(13) Stratagene, 1988, Bluescript Exo-Mung DNA Sequencing System instruction Manual, San Diego, California, U.S.A.

(14) The Qiagenologist, Application Protocols, 1988, Diagen, Dusseldorf, FRG.

(15) A.C. Forster, J. Mc Innes, D.C. Skingle and R. Symons, "Non-radioactive hybridization probes prepared by the chemical labelling of DNA and RNA with a novel reagent: Photobiotin" Nucl. Acis Res. (1985) 13: 745-763.

(16) E.D. Sverdlov, C.S. Monastyrskaya, L.I. Guskova, T.L. Levitan, V.I. Sheichenko and E.I. Budowsky, "Modification of cylidine residues with a bisulfite-O-methylhydroxylamine mixture", Biochim, biophys. Acta (1974) 340: 153-165.

(17) A.M. Poverenny, V.K. Podgorodnichenko, L.E. Bryskina, G.S. Monastyrskaya and E.D. Sverdlov, Mol. Immunol. (1979) 16: 313.

(18) M. Herzberg, A. Reinhartz, M. Ritterband, S. Twizer, N.I. Smorodinski and F. Fish, "New concept in diagnostic procedures based on non-radioactive DNA probes", Chimicaoggi, (Nov. 1987): 69-71.

(19) H.C. Birnboim, "A rapid alkaline extraction method for the isolation of plasmid DNA", Meth. Enzymol. (1983) 100: 243-270.

(20) J.J. Leary, D.J. Brigati and D.C. Ward, "Rapid and sensitive colorimetric method for visualizing biotin-labeled DNA probes hybridized to DNA or RNA immobilized on nitrocellulose: bio-blots", Proc. Natl. Acad. Sci., U.S.A., (1983) 80: 4045-4049.

(21) Bethesda Research Laboratories, Catalogue and Reference Guide (1988), Bethesda Research Laboratories, Gaithersburg, Maryland, U.S.A.

(22) Orgenics Chemiprobe, Instructions for Use, 1989. Orgenics, Yavne, Israel.

## Claims

1. Procedure for the detection of plant pathogens, performed totally or in part under field conditions, with the following features:

   a) A plant tissue, presumably containing a population of at least one phytopathogenic agent, is selected.

   b) From the chosen plant tissue, a localized sample dot is obtained on a detection support membrane made up of a membrane filter material able to immobilize the nucleic acids of the phytopathogenic agents.

   c) The nucleic acids eventually present in the applied sample (contained in the phytopathogenic agents of said population) are fixed onto the mentioned detection support membrane.

   d) The sample applied on the detection support membrane is put into close contact with a hybridization solution containing at least one probe complementary of the nucleic acids of at least one phytopathogenic agent; said at least one labeled probe containing an auxiliary reactive group.

   e) After hybridization to the probe, the detection support membrane is washed with a first washing solution, to remove the probe which did not hybridize in step d).

   f) The detection support membrane is treated with a blocking solution.

   g) The membrane is treated with at least one reagent of at least one solution until the auxiliary

24

reactive group (from the at least one probe) of the hybrid is bound to an enzyme (capable of catalizing the reaction of a chromogenic substrate to obtain a colored insoluble compound), by means of soaking the detection support membrane with said at least one solution of the at least one reagent.

h) The detection support membrane, after the above enzymatic labeling, is washed with a second washing solution.

i) The presence of hybrids in the localized sample is made visible, by means of a bath of the detection support membrane with a chromogenic substrate solution capable of producing, in the presence of said enzyme, an insoluble colored compound; said colored insoluble compound capable of producing in the localized sample a change in color visible with the naked eye, detecting the presence in the chosen tissue of a population of the at least one phytopathogenic agent.

2. Procedure for the detection of plant pathogens, according to claim 1 wherein
either the steps a) to i) are carried out under field conditions,
or the steps a) to e) are carried out under field conditions, while steps f) to i) are performed in the laboratory, with the intercalation between steps e) and f) of one drying step, one step consisting in carrying the detection support membrane from the field to a distant laboratory in a device suitable to maintain a dry atmosphere, and a rehydration step immediately prior to step f), the transport step including standing-by steps;
or the steps a) to c) are carried out under field conditions, while steps d) to i) are performed in the laboratory, with the intercalation between steps c) and d) of a step consisting in carrying at room temperature the detection support membrane from the field to a distant laboratory, the transport step including standing-by steps.

3. Procedure for the detection of plant pathogens, according to claim 1, with the characteristic that the probe referred to in step d) is labeled by sulfonation of the cytosines, said at least one solution of the at least one reagent of step g) being constituted:
either by a first solution of a first reagent, a monoclonal antibody directed against DNA modified by sulfonation and by a second solution of a second reagent, a specific antibody against the mentioned monoclonal antibody; said specific antibody being conjugated with said enzyme; step g) being carried out by a first step of soaking in said first solution, incubating at room temperature, a second step of washing with a second washing solution, a third step of draining and a fourth step of incubating in the second solution at room temperature;
or by a solution of a reagent, a monoclonal antibody directed against DNA modified by sulfonation; being said monoclonal antibody conjugated with said enzyme; step g) being carried out by the single step of soaking in said solution, incubating at room temperature.

4. Procedure for the detection of plant pathogens, same as claimed in 1, with the characteristic that the probe of step d) is labeled with biotin groups; said at least one solution of the at least one reagent of step g) being constituted:
either by a first solution of a first reagent, avidin and by a second solution of a second reagent, constituted by the mentioned enzyme with covalently bound biotin; carrying out step g) by a first step of soaking in said first solution, incubating at room temperature, a second step of washing, a third step of draining and a fourth step of incubating in the second solution at room temperature;
or by a first solution of a first reagent, streptavidin, and by a second solution of a second reagent, constituted by the mentioned enzyme with covalently bound biotin; carrying out step g) by a first step of soaking in said first solution, incubating at room temperature, a second step of washing, a third step of draining and a fourth step of incubating in the second solution at room temperature;
or by a single solution of an avidin-enzyme complex; carrying out step g) by the sole step of soaking in said single solution, incubating at room temperature;
or by a single solution of a streptavidin-enzyme complex; carrying out step g) by the sole step of soaking in said single solution, incubating at room temperature.

5. Procedure for the detection of plant pathogens, according to claim 1, with the characteristic that in step b) the localized sample is obtained:
either by squashing the target tissue, pressing it with a hard device against the detection support membrane;
or by a first step of cutting the target tissue and an immediate second step of localized imprinting

25

the exudate of the cut area on the detection support membrane;

or by a first step of crushing the target tissue, a second step of centrifuging, a third step of decanting the clarified supernatant and a fourth step of applying an adequate volume of this supernatant onto the detection support membrane.

6. Procedure for the detection of plant pathogens, according to claim 5, with the characteristic that in said first crushing step an extraction solution is added, to regulate the pH and denature nucleic acids degrading enzymes, and possibly between the third step of decanting the supernatant and the fourth step of applying it onto the detection support membrane, a step is introduced for the purification of the nucleic acids.

7. Procedure for the detection of plant pathogens, according to claim 1, with the characteristic that before step b) the detection support membrane is wetted with a pretreatment solution capable of preventing the degradation of the nucleic acids by enzymes released from the sample.

8. Procedure for the detection of plant pathogens, according to claim 1, with the characteristic that the detection support membrane used in step b) is selected from a nitrocellulose membrane filter, a nylon membrane filter, and a modified nylon membrane filter.

9. Procedure for the detection of plant pathogens, according to claim 1, wherein step c) of nucleic acids fixing is performed:

either by natural drying of the detection support membrane at room temperature;

or by heating (baking) the detection support membrane;

or by baking the detection support membrane in a microwave oven;

or by irradiation of the detection support membrane with ultraviolet light.

10. Procedure for the detection of plant pathogens, according to claim 1, wherein when the detection support membrane in step (b) is a modified nylon membrane filter, step c) of fixing the nucleic acids is performed by a first step of soaking the detection support membrane in an alkaline solution, such as 0.5 M NaOH, and a second step of washing with a neutralizing solution, such as 0.1 M Tris. HCl, 1 M NaCl, pH 7.5.

11. Procedure for the detection of plant pathogens, same as claimed in claim 1, with the characteristic that prior to step d), the detection support membrane is submitted to a prehybridization step, consisting in soaking it for 20 to 30 minutes in a prehybridization solution at a temperature between 37 and 42° C.

12. A set of reagents for the detection of plant pathogens performed totally or in part under field conditions comprising:

a) a detection support membrane constituted by a membrane filter capable of immobilizing the nucleic acids of the phytopathogenic agents;

b) at least one hybridization solution containing at least one probe complementary to the nucleic acids of at least one phytopathogenic agent, and labeled with an auxiliary reactive group;

c) a first washing solution:

d) a blocking diluent;

e) a blocking powder to be added to the blocking diluent at the time of use to obtain a blocking solution;

f) at least the solution of the at least one reagent to bind an enzyme to the auxiliary reactive group of the presumably formed hybrid;

g) a second washing solution; and

h) a chromogenic substrate solution.

13. Set of reagents for the application of the procedure claimed in claim 12, with the characteristic that said at least one probe of the at least one hybridization solution b) is selected from a monomeric probe, and a dimer of a monomeric probe.

14. Set of reagents for the application of the procedure claimed in claim 12, with the characteristic that said at least one probe of the at least one bybridization solution b) has an additional tail of several sulfonated cytosines covalently linked to one end of the probe DNA strand.

15. Set of reagents for the application of the procedure claimed in claim 12, with the characteristic that said detection support membrane is selected from nitrocellulose, nitrocellulose on a structural support, nylon, and modified nylon.

16. Set of reagents for the application of the procedure claimed in claim 12, with the characteristic that the composition of said hybridization solution is selected from:

(i) a solution of formamide 50%, sodium chloride 450 mM, sodium acid phosphate 25 mM, EDTA 2.5 mM, sodium dodecyl sulfate 1%, sodium pyrophosphate 0.001%, sodium polyanetholesulfonate 0.1%, labeled probe 0.1 $\mu$g/ml; and

(ii) a solution of guanidine thiocyanate 2 to 4 M sodium chloride 300 mM, trisodium citrate 30 mM, poly(acrylic)acid (molecular weight 90,000, neutralized) 1.5%, sarcosine 1%, EDTA 60 mM, salmon sperm DNA denatured by sonication 100 $\mu$g/ml, labeled probe 0.1 $\mu$g/ml, pH: 7.5.

17. Set of reagents for the application of the procedure claimed in claim 12, with the characteristic that said washing solution is selected from:

(i) a solution containing sodium chloride 150 mM, trisodium citrate 15 mM, sodium dodecyl sulfate 0.1%, pH: 7.0, and

(ii) a solution containing sodium chloride 15 mM, trisodium citrate 15 mM, sodium dodecyl sulfate 0.1% pH: 7.0.

18. Set of reagents for the application of the procedure claimed in claim 12, with the characteristic that said blocking diluent is selected from:

(i) a solution containing sodium chloride 25 mM, Tris. HCl (pH:7.5) 50 mM, EDTA 1 mM, Tween 20 0.3%, and

(ii) a solution containing sodium chloride 25 mM, Tris. HCl (pH:7.5) 50 mM, EDTA 1 mM, Tween 20 0.3%, Heparin (Na) 3.5 mg/ml.

19. Set of reagents for the application of the procedure claimed in claim 12, with the characteristic that said blocking powder is constituted by skimmed milk 91 g and bovine seroalbumin (fraction V) 9 g.

20. Set of reagents for the application of the procedure claimed in claim 12, with the characteristic that said second washing solution contains sodium chloride 0.5 M and Tween 20 0.1%.

21. Set of reagents for the application of the procedure claimed in claim 12, with the characteristic that the solution of chromogenic substrate is constituted by:
- a diluent composed by Tris. HCl 100 mM (pH: 9.5), NaCl 100 mM, $MgCl_2$ 5 mM;
- 3 mg of a mixture of nitro-blue-tetrazolium + glucose (1 part + 9 parts by weight, respectively);
- 4 $\mu$l of a 50 mg/ml solution of 5-bromo-4-chloro-3-indolyl phosphate in N,N-dimethylformamide.